# EUROPEAN PATENT APPLICATION

(11) **EP 3 695 843 A1**
(43) Date of publication of application: **19.08.2020**
(21) Application number: 19157526.5
(22) Date of filing: 15.02.2019
(51) Int. Cl.: A61K 31/695, A61K 31/80, A61K 33/06, A61K 33/00, A61P 21/02

(54) **MEDICAMENT FOR THE PREVENTION AND TREATMENT OF MUSCLE CRAMPS**

(71) Applicant: Bio Minerals N.V., 9070 Destelbergen (BE)
(72) Inventor: COOLSAET, Boudewijn Louis René André, 2990 Wuustwezel (BE); VAN VOOREN, Christianne Augusta Adolf, 2990 Wuustwezel (BE)
(74) Representative: Nollen, Maarten Dirk-Johan

(57) **Abstract**

Pharmaceutical composition comprising a pharmaceutically effective amount of bioavailable silicon in a daily dose of at least 3 mg elemental silicon, and a pharmaceutically effective amount of a magnesium compound in a daily dose of 50-500 mg elemental magnesium. The composition is used for prevention, inhibition and/or treatment of muscle cramps, such as skeletal muscle cramps.

## Description

### FIELD OF THE INVENTION

The invention relates to a medicament for prevention, inhibition and treatment of muscle cramps. The invention further relates to a method of prevention, inhibition and treatment of muscle cramps by administration of a medicament.

### BACKGROUND OF THE INVENTION

Muscle cramps are involuntary, generally painful contractions of a muscle or muscle group. Cramps may occur in a skeletal muscle or in smooth muscle. Some patients are bothered by very frequent and severe muscle cramps that may be disabling. A cross-sectional prevalence study of 365 outpatients aged 65 or older in the United Kingdom reported that 50% of outpatients report frequent cramps (Abdulla AJ et al. Int J. Clin Pract 53(1999), 494-496.) Another review of 515 elderly veterans report a similar prevalence of 56%, with half having cramps occurring at least once a week (Oboler SK et al, Muscle Nerve 17(1994), 1243-1249).

Skeletal muscle cramps often occur during night, and is considered a sleep-related movement disorder in the category of the International Classification of Sleep Disorders (ICSD-2). In this classification system, nocturnal leg cramps are termed 'sleep-related leg cramps' and are consistent with the International Classification of Diseases (ICD-9) code 327.52, which has the following definition: a painful sensation in the leg or foot is associated with sudden muscle hardness or tightness indicating a strong muscle contraction and the painful muscle contractions in the legs or feet occur during the sleep period, although they may arise from either wakefulness or sleep. Forceful stretching relieves the pain of the affected muscles and leg cramps are not explained by another current (medical) sleep disorder or medication use. Besides the legs and feet, cramps of striated muscles can occur in all parts of the body including other limps (hands, arms), the diaphragm and the pelvic floor.

Smooth muscle contraction may be due to menstruation or gastroenteritis. The latter can cause oesophageal spasm, i.e. a contraction of the oesophagus, and spasms of the stomach and the intestine.

Several medications have been proposed for the prevention, inhibition or treatment of such recurrent muscle cramps. Only one medication has been proven clinically to be effective, namely quinine, but common and serious side effects of quinine have been reported in the literature. Therefore, the use of quinine or quinine derivatives is not recommended for routine treatment of cramps (HD Katzberg et al, Neurology 74(2010), 691-696). Gabapentin was tested in a Class I clinical study, but no difference was found between treatment and placebo with respect to any symptom score. Another study found no effect on the mean number of cramps, number of nights with cramps or sleep disturbance, when the medication naftidrofuryl oxalate was administered. A Class II study using magnesium citrate (900 mg) could not conclude that there was a significant improvement in the number of cramps in patients on treatment, and had a high dropout rate. Another Class II study evaluating the efficacy of magnesium sulphate (300 mg dose) found that treatment was not superior to placebo for number of cramps, severity, duration or sleep disturbance. A class-II study on diltiazem hydrochloride found no effect on the intensity of cramps. And although agents such as baclofen, carbamazepine and oxcarbazepine are frequently used in clinical practice for the management and treatment of muscle cramps, there are no clinical trials in the literature evaluating their efficacy for this indication (Katzberg et al, ibid).

Therefore, an effective treatment for recurrent muscle cramps, for instance skeletal muscle cramps, is highly desired, as many people suffer from such muscle cramps, and often frequently and over long periods, leading moreover to sleep disturbance and intense pain.

### SUMMARY OF THE INVENTION

It is therefore an object of the invention to provide a medicament for use in the prevention, inhibition or treatment of muscle cramps.

It is a further object to provide a medicament for use in the prevention, inhibition and/or treatment of recurrent muscle cramps, and more particularly muscle cramps for which no underlying cause can be identified, i.e. idiopathic muscle cramps.

It is again a further object to provide a method of prevention, inhibition or treatment of such muscle cramps.

According to a first aspect of the invention, a pharmaceutical composition of matter is provided, comprising a pharmaceutically effective amount of bioavailable silicon, and a pharmaceutically effective amount of a bioavailable magnesium compound for use in prevention, inhibition or treatment of muscle cramps.

According to a second aspect of the invention, a pharmaceutical composition of matter is provided comprising a pharmaceutically effective amount of bioavailable silicon in a daily dose of at least 3 mg elemental silicon, and a pharmaceutically effective amount of a magnesium compound in a daily dose of 50-500 mg elemental magnesium.

According to a third aspect of the invention, a pharmaceutical combination of a pharmaceutically effective amount of bioavailable silicon in a daily dose of at least 3 mg elemental silicon, and a pharmaceutically effective amount of a magnesium compound in a daily dose of 50-500 mg elemental magnesium, for use in prevention, inhibition or treatment of muscle cramps.

According to a fourth aspect of the invention a single pharmaceutical composition comprising a bioavailable silicon compound and a bioavailable magnesium compound is provided, preferably for use in the prevention, inhibition or treatment of muscle cramps, and preferably comprising an pharmaceutically effective amount of bioavailable silicon in a daily dose of at least 3 mg elemental silicon and further comprising a pharmaceutically effective amount of a magnesium compound in a daily dose of 50-500 mg elemental magnesium.

According to a fifth aspect of the invention, a pharmaceutical composition of matter is provided, comprising a pharmaceutically effective amount of bioavailable silicon, preferably in a daily dose of at least 3 mg elemental silicon, and a pharmaceutically effective amount of a magnesium compound, preferably in a daily dose of 50-500 mg elemental magnesium, for the prevention, inhibition or treatment of skeletal muscle cramps, and most preferably for the inhibition or treatment of recurrent muscle cramps.

According to a sixth aspect of the invention, a pharmaceutical composition of matter is provided, comprising a pharmaceutically effective amount of bioavailable silicon in the form of a silicon compound chosen from orthosilicic acid and/or an oligomer thereof, which silicon compound is provided with a stabilizing agent inhibiting polymerisation of the silicon compound, wherein the stabilizing agent preferably comprises or is a quaternary ammonium compound and wherein most preferably the stabilizing agent is choline or a choline compound, wherein the pharmaceutical composition is for the prevention, inhibition or treatment of muscle cramps, such as recurrent muscle cramps, for instance skeletal muscle cramps.

According to a seventh aspect of the invention, a method of prevention, inhibition or treatment of muscle cramps is provided, wherein bioavailable silicon and magnesium are orally administered, wherein preferably the bioavailable silicon is administered at a daily dose of at least 3 mg elemental silicon, wherein preferably the magnesium is administered at a daily dose of 50-500 mg elemental magnesium.

The inventors have surprisingly found that the combination of bioavailable silicon and magnesium is effective in relation to muscle cramps. That relates to the prevention of muscle cramps, but also to the inhibition and treatment of muscle cramps. Use is herein made of a combination of silicon and magnesium is mutually adjusted daily doses that are pharmaceutically effective. Though it has been stated that silicon may affect the absorption, retention or action of other mineral elements (e.g., aluminium, copper, magnesium, (FH Nielsen, J Trace Elements in Medicine and Biology, 28(2014), 379-382), the efficacy on treatment of muscle cramps, and recurrent muscle cramps in particular, comes as a surprise. According to Katzberg et al (ibid), muscle cramps are caused by ectopic discharges from nerves or nerve terminals; therefore a variety of neuropathic conditions are commonly associated with cramps. While silicon is known to have a beneficial effect on bone and connective tissue health, the efficacy in combination with magnesium to prevent, inhibit or treat recurrent muscle cramps, when administered orally and more preferably daily, cannot be derived thereof. It is particularly relevant for the striated muscle tissue.

The term 'bioavailable silicon' is used in the context of the present application to refer to silicon-containing preparations that are absorbed by the human body. Such silicon-containing compounds are preferably silicic acids, though the use of synthetic alkyl-silanols is not excluded. In the medicament, the silicic acid may be polymerized but merely to an extent that hydrolysis thereof into monomers, dimers and trimers is feasible in the gastro-enteric tract.

The term 'pharmaceutical composition of matter' is deemed to cover in the context of the present invention any form wherein the active ingredients (bioavailable silicon and the - bioavailable - magnesium compound) are suitable for administration, either concomitant administration, or time-delayed administration, and/or administration via different routes. According to a first and preferred embodiment of the pharmaceutical composition of matter, the bioavailable silicon and the magnesium compound are part of a single pharmaceutical formulation. A most preferred implementation hereof is a formulation wherein the bioavailable silicon and the magnesium compounds are present as a solid mixture. However, further implementations are feasible and not excluded. According to a second embodiment of the pharmaceutical composition of matter, the composition is subdivided over more than one formulation, such as one formulation comprising bioavailable silicon (as primary and/or sole active ingredient) and/or one formulation comprising the magnesium compound (as primary and/or sole active ingredient). As will be discussed further in more detail, other subdivisions are feasible as well.

Preferably, the silicon compound is used in combination with a stabilizing agent inhibiting polymerization of the silicon compound. The inhibition is more particularly such that the formation of water insoluble and no longer hydrolysable silicon polymers is prevented. Such silicon polymers are not bioavailable, i.e. consumption thereof does not allow the body to absorb silicon in a form ready for absorption, which is particularly the silicic acid monomer orthosilicic acid, oligomers thereof and presumably certain variations thereto, such as silanols and silicates. Stabilization can be obtained by use of amino acids, other organic acids such as salicylic acid, sorbitol acid, ascorbic acid, lactic acid and caproic acid, peptides and protein hydrolysates, carnitine, phenolic or polyphenolic compound such as vanillin (4-hydroxy-3-methoxybenzaldehyde), quaternary ammonium compounds, such as betaine and choline and derivatives thereof. Preferably, the stabilizing agent is a choline compound as the stabilizing agent, and the choline compound is chosen from the group of choline, choline hydroxide, acetylcholine, betaine, glycerophosphorylcholine, sphingomyelin, phosphatidylcholine, lecithin or a salt thereof.

The choline compound is preferred as a stabilizing agent, and is for instance chosen from choline chloride, choline bitartate, choline hydroxide, choline dihydrogen citrate, choline 2-4-dichlorophenoxyacetate (2,4 D choline salt), choline acetate, choline carbonate, choline citrate, choline tartate, choline lactate, choline dibutyl phosphate; choline O,O'-diethyl dithiophosphate, choline dihydrogen phosphate; choline phosphate. Good results have been found with choline chloride. The choline compound is preferably present in a concentration of at least 20wt% of a liquid formulation of the silicon compound. It is deemed an advantage of the use of choline as a stabilizing agent is that it may have a synergetic effect on muscles. Choline acts as a partial agonist for a neurotransmitter acetylcholine in neuromuscular junction. Choline is converted to acetylcholine by the enzyme choline acetylase, and can then be used as a neurotransmitter. Hence, the combination of magnesium, bioavailable silicon and choline may positively effect both the muscle as well as the nerves involved in muscle contraction.

The bioavailable silicon can be generally specified as a compound of the formula YₓSi(OH)₄₋ₓ or an oligomer thereof, wherein Y is optionally substituted (C₁-C₄)alkyl, (C₂-C₅)-alkenyl, (C₁-C₄)-alkoxy, amino, and wherein x is 0-2.

In a most preferred embodiment, the silicic acid compound is the silicic acid monomer known as orthosilicic acid, and/or oligomers thereof. This corresponds to a value x=0 in formula (I). The bioavailability of orthosilicic acid has been proven directly in a plurality of scientific studies, which has also been acknowledged by the European Food Safety Authority (EFSA). It is moreover a natural ingredient as compared to monomethyltrisilanol, which does not occur in nature but is a man-made synthesized compound.

Preferably, in the invention, the silicic acid substantially comprises oligomers and/or monomers of orthosilicic acid. The oligomers are for instance oligomers comprising less than 1000 monomers, preferably less than 100 monomers per molecule. More preferably, the oligomers are such that at least 80% and preferably at least 90% of the silicon atoms are herein bonded to at most 3 other silicon atoms via a silicon-oxygen-silicon bridge. The term substantially herein suitably refers to at least 95wt%, preferably at least 98wt%, more preferably at least 99wt%. Silicic acid preparations that are polymerized merely to an extent that hydrolysis thereof into monomers, dimers and trimers is feasible in the gastro-enteric tract, are called bioavailable silicon. Silicic acid of such preparations can be absorbed by the human body.

In an alternative embodiment, use is made of a trisilanol compound as the silicon compound, such as monomethyltrisilanol. This corresponds to the option of x=1 in formula I. The trisilanol compound may further be used in a blend of silicon compounds according to formula (I) with x=0-2, and preferably x = 0 or 1. One preferred side group Y is C1-C4 alkyl, such as methyl.

The silicon compound, such as the silicic acid compound, and the stabilizing agent, such as the choline stabilizing agent are preferably provided in a mutual weight ratio of stabilizing agent/silicon compound of 1-5, such as 2-4.

The bioavailable silicon is preferably administered at a daily dose of at least 3 mg/day, more preferably at least 5 mg/day. Most preferably, the daily dose of silicon is at most 15 mg/dose, based on elemental silicon. It has been found that a relatively high silicon concentration is well-tolerated and is a secure dose that can be administered during longer periods, without any adverse effect.

The magnesium is more particularly a magnesium (Mg) salt. Examples include magnesium sulphate, magnesium lactate, magnesium aspartate hydrochloride, magnesium oxide, magnesium citrate, magnesium bicarbonate, magnesium phosphate, magnesium chloride, magnesium gluconate. Preferably, at least one organic, chelated magnesium salt is used as the magnesium salt. Examples thereof include aspartate, arginate, ascorbate, citrate, gluconate, lactate, picolinate, taurate, glycerophosphate, bisglycinate, malate, piccolinate salts of magnesium. It is deemed more preferable that more than one organic salt is used. This is considered advantageous for the adsorption of magnesium into the bloodstream and because the organic salts have less side effects. The magnesium is preferably applied at a daily dose in the range of 50-500 mg, for instance 100-400 mg, such as 200-300 mg and 350- 400mg.

Most preferably, the doses of silicon and magnesium are amended in function of the age, sex and muscular development of a patient. Furthermore, a patient have a larger muscular mass, such as an athlete or other sport professional (for instance in football, cycling, iceskating, hockey, swimming) may take a larger dose, for instance a double dose.

The pharmaceutical composition or combination is preferably used on a regular basis so as to prevent or inhibit occurrence of recurrent muscular cramps that otherwise would occur frequently, for instance once or more often per week. The regular basis is most preferably daily.

In an alternative embodiment, the pharmaceutical combination is used as a rescue medication, i.e. for use to treat muscle cramp. A preferred dose in such a situation is 10 mg up to 20 mg elemental silicon and 200 mg up to 400 mg magnesium.

The pharmaceutical combination and the pharmaceutical composition are particularly used for the prevention, inhibition or treatment of muscle cramp in humans and animals. Muscle cramps are not merely relevant for human beings, but also for other animals and particular mammals. Examples of animals for which the present invention is deemed to have a positive effect include horses, cows, pigs and dogs.

The pharmaceutical combination and the pharmaceutical composition can be applied for the prevention, inhibition and/or treatment of muscle cramps both in smooth muscles and in skeletal muscles. Examples of cramp in smooth muscles include indications such as detrusor instability, and/or micro-motions in the bladder, typically occurring as a sign of or as part of detrusor instability. Another example of cramps in smooth muscles are intestinal spasms. Furthermore, contraction in muscles surrounding the urethra may be addressed with the composition or combination of the invention.

Examples of skeletal muscle cramp are widespread through the body, and for instance may occur in extremities, such as the limbs, including hands, feet, arms and legs. Skeletal muscles are distinct from other muscles, i.e. smooth muscles and cardiac muscles and are also known as voluntary muscles as they are under control of the brain. The skeletal muscles have a different morphological structure contrarily to smooth muscles.

In an important embodiment, the combination or composition is used for inhibition or treatment of recurrent skeletal muscle cramps. The term 'recurrent muscle cramp' is used for muscular cramps that occur regularly, such as several times per year, at least 3 times per month, at least once a week or even more frequently, such as almost every day or night. The inhibition or treatment according to this embodiment involves regular administration. The consequence of the treatment of patients with recurrent skeletal muscle cramp will be that patients regularly and preferably daily take the pharmaceutical composition or combination comprising bioavailable silicon and bioavailable magnesium. Therewith, the occurrence of muscle cramps can be prevented or at least largely suppressed.

In another embodiment, the combination or composition is used for prevention, inhibition or treatment of muscle cramps (such as skeletal muscle cramps) during revalidation after an operation. Clinical tests have proven that the invention is effective for such an indication. Herein, the bioavailable silicon and bioavailable magnesium are preferably administered during a predefined period of revalidation, for instance in the range of two weeks to 1 year, preferably one month to six months, and in this period daily.

A daily dose of up to 250 mg magnesium per day is currently deemed preferred for use in the prevention of skeletal muscle cramps. This is more preferably used in combination with a daily dose of up to 10 mg elemental silicon per day. A suitable daily dose is for instance 50-200 mg/day magnesium in combination with at least 5 mg elemental silicon per day. Herein, the elemental silicon is most preferably applied in the form of a silicic acid compound that is combined with a stabilizing agent, for instance a choline compound. The daily magnesium dose is more particularly chosen as 30-70wt% of a daily reference intake of magnesium. Preferably, the dose is in the range of 40-60wt% of the said daily reference intake.

A lower dose is not excluded. It is believed by the inventors as one possible explanation, that the cause of muscular cramps is not just or dominantly the availability of magnesium in food and/or its adsorption from the gastro-intestinal tract into the blood, but rather the transmission of the magnesium into muscular cells. The silicon herein is deemed to play a key role. In an important embodiment, the silicon compound is stabilized with a choline compound as stabilizing agent, wherein the choline compound further contributes to the transport of magnesium into muscular cells. As a consequence, the magnesium dose may be relatively low in comparison with the daily reference intake.

For the treatment of skeletal muscle cramps, and particularly of recurrent skeletal muscle cramps, a higher magnesium dose is preferred, for instance 150-500 mg per day. In this situation, the preferred daily magnesium dose is at least 50wt% of a daily reference intake of magnesium and more preferably at least 75wt% or eve at least 100wt% of the said daily reference intake of magnesium. The silicon dose is preferably at least 5 mg/day for this specific indication.

Suitable daily doses include 100 mg magnesium in combination with 4 mg silicon; 250 mg magnesium in combination with 6 mg silicon and 400 mg magnesium in combination with 12 mg silicon. All these doses are based on elemental magnesium and silicon. More generally, the mutual weight ratio of the elemental magnesium and elemental silicon daily doses is preferably in the range of 20-50.

For sake of clarity, in the context of the present application, the term 'treatment' refers to the treatment of an actual disease, disorder or other medical condition, and therewith including also attempts thereto that would suppress or relief. The term 'prevention' refers to any action for the avoidance of a disorder, and any action that would counteract or prevent generation of symptoms. Curing refers to any action that leads to recovery such that no further medication or treatment is necessary.

In a preferred embodiment, the pharmaceutical composition of matter is provided as a single pharmaceutical formulation. This has the advantage of user compliance, so as to ensure that both the components, i.e. the bioavailable silicon and the magnesium are administered by a user in the prescribed ratio between bioavailable silicon and magnesium.

In a preferred implementation thereof, the bioavailable silicon comprises a silicon compound that is provided with a stabilizing agent inhibiting polymerisation of the silicon compound and with a carrier material, said combination of silicon compound, stabilizing agent and carrier being in granulate form, wherein the magnesium is in solid form and the granulate and the magnesium are present in the pharmaceutical formulation as a solid mixture. It has been found by the inventors in experiments that such a formulation can be prepared relatively easily and is user-friendly. In one embodiment thereof, the binder is water-soluble. Then, a user may dissolve the formulation in water or another drink prior to administration thereof.

When implemented as a single pharmaceutical formulation, the daily dose may be achieved by oral administration of one or more individual tablets, drops, capsules or other dosage forms. It is deemed preferable that the daily dose is divided over a plurality of individual tablets or capsules, for instance two or four, further in dependence of the daily dose of silicon and magnesium.

As a further preference, the granulate formulation of the bioavailable silicon is particulate wherein at least 80wt% of the particles has a size in the range of 100-800 µm, as measured by means of sieve analysis. Sieve analysis of a preferred example indicates the feasibility to arrive at a distribution such that 90 % of the granules are smaller than 600 µm. Optionally, sieving may be applied to select a fraction of granules with a specific, narrow particle size distribution or to remove particles with a size > 600 µm. The silicon concentration of the dried granulate is higher than 0.5 % w/w. More preferably, the particle size distribution is such that a fraction with particles smaller than 100µm accounts for at most 15wt% of the particles, more preferably at most 10wt% of the particles or even at most 5wt% of the particles.

In a preferred embodiment, the generated granules are dried subsequent to their formation to a predefined degree of moisture. Such a degree of moisture is for instance at most 5wt%, although a moisture level of at most 4wt% or at most 3wt% could be chosen alternatively.

According to an important embodiment, the granulate is a fluidized granulate. More particularly, it is obtainable by the method comprising the steps of (1) providing a liquid formulation of the silicon compound; (2) Introducing a particulate carrier into a fluidized bed granulator, and (3) Spraying said liquid formulation into the fluidized bed granulator during operation thereof, wherein carrier particles agglomerate to granules with the liquid formulation acting as a binder. The fluidized bed granulation process ensures a uniform distribution of the liquid silicon formulation through the carrier material. Furthermore, good size distributions have been found, and the fluidization process results in granulates with a relatively low density and porosity, which is deemed advantageous for the dissolution (including the generation of a colloidal solution, which is formally a - dilute - suspension). This embodiment is particularly important in combination with the use of orthosilicic acid and/or oligomers as the silicon compound and the presence of a stabilizing agent for the silicon compound, and more particularly a choline compound as the stabilizing agent. It has turned out more difficult to create a granulate with uniform granule size with other wet granulation methods for this specific silicon compound with its stabilizing agent.

In a preferred embodiment, the granulate has a density in the range of 0.25-060 g/cm³, more preferably 0.30-0.55 g/mol, such as 0.33-0.36 g/cm³ or 0.40-0.53 g/mol. This density is clearly lower than densities obtained with extrusion-spheronisation as this typically results in a density above 0.75 g/cm³.

In a preferred implementation, the drying step occurs in the fluidized bed granulator in which the granules are generated. The 3 different steps of spraying, granulation and drying can be performed by fluidized bed granulation, and are in one implementation performed in a single granulator unit. Such fluidized bed granulation units are known per se, for instance from Glatt, with a variety of accessories for different process steps. The fluidized bed granulation unit usually comprises a spray nozzle that can be adjusted as to position and spray rate. Preferably, use is made of a so-called top nozzle. Suitable spray rates are in the range of 500-2000 g/min.

In a further embodiment, the obtained granules may be provided with a coating, which is preferably applied by fluidized bed granulation. If so desired, such a coating may be applied in the same fluidized bed granulation process in a single unit. The coating can be done to change the flavor or the aroma, to protect against oxidation, to change the visual appearance or for enteric coating. Examples of coatings are film coatings, enteric coatings delayed release coatings, hot melt coatings.

In again a further embodiment, the fluidized bed granulation is carried out with a carrier gas, preferably air, which is heated to above room temperature. A preferred temperature of the carrier gas is at least 50°C up to 120°C, and preferably in the range of 70-100°C. The heated carrier gas further leads to drying of the material. In a preferred embodiment 25-40wt% stabilized silicic acid is sprayed on 60-75 wt% carrier material, such as modified starch, for instance esterified starch. Several types of modified starch can be combined to alter the particle size distribution of the obtained granulate. A preferred ratio is 1:2 between the liquid stabilized silicic acid formulation and the carrier material.

Preferred carrier materials include one or more chemically modified starch material, such as dextrin, acid-treated starch, alkaline-modified starch, bleached starch, oxidized starch, enzyme-treated starch. A preferred class of modified starch materials are the esterified starches, such as monostarch phosphate, distarch glycerol, distarch phosphate esterified with sodium trimetaphosphate, phosphated distarch phosphate, acetylated distarch phosphate, starch acetate esterified with acetic anhydride, starch acetate esterified with vinyl acetate, acetylated distarch adipate, acetylated distarch glycerol, starch sodium octenyl succinate and/or combinations thereof. Hydroxypropyl starch, hydroxypropyl distarch phosphate, hydroxypropyl distarch glycerol may also be suitable. The modified and esterified starches may further be used in the form of salts, such as sodium salts.

In a further embodiment, the composition further includes one or more further nutrients and vitamins, more preferably vitamins, such as vitamin B6, B9, B12, vitamin D and taurine. These vitamins tend to support the digestion of the magnesium and contribute to good health.

In an alternative embodiment, the pharmaceutical composition of matter is supplied as a kit of parts. In one embodiment hereof, a first part of the kit is a formulation comprising the bioavailable silicon and the second part of the kit is a formulation comprising the bioavailable magnesium. In an alternative embodiment, a first part of the kit is a formulation comprising both bioavailable silicon and bioavailable magnesium and a second part of the kit is a formulation comprising either bioavailable silicon or bioavailable magnesium. The kit of part of this embodiment enables a variation in the ratio between silicon and magnesium to meet specific patient groups and/or clinical situations. Administration in different parts furthermore facilitates the administration of a first part via a different administration route than a second part.

Administration of the bioavailable silicon and/or the bioavailable magnesium may be done via different administration routes, including oral, rectal, intramuscular, intravenous and sublingual. In the event of intramuscular and/or intravenous administration of bioavailable silicon, use is preferably made of a silanol compound. In the event of administration of choline-stabilized silicic acid monomer and oligomers, oral administration is highly preferred.

It is observed that any galenic form is feasible for the pharmaceutical composition comprising bioavailable silicon and bioavailable magnesium, and/of for compositions comprising either bioavailable silicon or bioavailable magnesium. Examples include liquids - such as a syrup, drops, a drinkable solution, capsules (among which liquid-filled capsules and capsules filled with solid particles), tablets, water-soluble tablets, powders and other water soluble formulations to be administered after dissolution and/or dispersion into water or an aqueous composition such as milk or a juice or a sweet drink and the like. Formulations for intravenous, intramuscular and rectal are known per se to the skilled person.

In again a further embodiment, the bioavailable silicon and the bioavailable magnesium are supplied for integration into food and/or feed, such as for instance an animal feed composition. Also for administration to human beings, integration into food products is not excluded, for instance as part of a sports drink.

In the event of supplying the combination of bioavailable silicon and bioavailable magnesium as a kit of parts, a combined packaging may be used to ensure that patients take both parts in the required combination. Such a combined packaging is furthermore deemed beneficial for patient compliance.

It is observed that any of the preferred options discussed above are applicable to all aspects of the invention, also when not discussed explicitly.

These and other aspects of the invention will be further elucidated with reference to the examples.

### EXAMPLE 1

A male patient at an age of 79 years had recurrent muscle cramps during a period of 23 years. Muscle cramps occurred in skeletal muscles in hands, lower arms and legs, as well as in intercostal muscles. The patient was otherwise healthy, did not take any other medications, did not smoke and used coffee and alcohol as common, without being addicted thereto. Muscle cramps occurred during the day as well as during the night, at a frequency of several cramps per day. As a consequence, the muscle cramps prohibited working as a doctor and further prohibited swimming or any other sport. The patient had been subjected to a therapy of magnesium (as a salt), in a daily dose of 450 mg per day, which was not effective and did not provide any relief or inhibition of the muscle cramps.

The patient thereafter switched to a therapy comprising on a daily basis 10 mg stabilized silicic acid, wherein the silicic acid was in the form of orthosilicic acid and/or oligomers. The stabilizer was choline, which was administered at a daily dose of 200 mg. This combination is sold as Biosil™ and is commercially available from Bio Minerals N.V., Destelbergen, Belgium. Use was made of the solid form of Biosil™. Two capsules per day were administered. The magnesium dose was 450 mg, and was in the form of magnesium phosphate, magnesium citrate, magnesium bicarbonate. It was a dosage form of magnesium commercially supplied as Promagnor™ by Merck Consumer Healthcare nv, Overijse, Belgium.

The patient was free of cramps as of the night following the day at which he started with the therapy.

### EXAMPLE 2

The patient of Example 1 continued therapy with the combination of bioavailable silicon and magnesium in the said daily doses for a period of 1 year. He remained free of cramps.

### EXAMPLE 3

A 59-year old person underwent a foot operation to straighten the position of two toes and shorten two other toes. During revalidation after the operation, the person got muscle cramps in the operated foot. The muscle cramps occurred daily, in the course of the day and much more when the foot was tired. Furthermore, certain foot positions, such as those necessary for putting on socks and shoes, led to muscle cramps.

This person was given the combination of bioavailable silicon in the form of Biosil™ in solid form (capsules) and magnesium in the form of Promagnor™. The daily doses were 10 mg Silicon (elemental weight) and 450 mg Magnesium (elemental weight). The choline stabilizer was therewith administered at a daily dose of 200 mg. Thereafter, cramps disappeared.

### EXAMPLE 4

An 80-year old person had nocturnal muscle cramps during several years, leading to significant sleep disturbance and related tiredness. He was administered bioavailable silicon as Biosil™ in solid form and magnesium as Promagnor™ The daily doses were 10 mg Silicon (elemental weight) and 450 mg Magnesium (elemental weight). The choline stabilizer was therewith administered at a daily dose of 200 mg. The cramps disappeared completely.

### EXAMPLE 5

A 52-year old person employed as a nurse faced several muscle cramps during the day and at night. Furthermore, the person had issues with the joints. She was administered bioavailable silicon as Biosil™ in solid form and magnesium in the form of Promagnor™ The daily doses were 10 mg Silicon (elemental weight) and 450 mg Magnesium (elemental weight). The choline stabilizer was therewith administered at a daily dose of 200 mg. The person was free of cramps after a treatment of 10 days. Joint pains disappeared as well.

### EXAMPLE 6

A 43-year old person being a nurse got cramps during running (sport). The cramps started after running about 2 km. The cramps occurred only during sport, hence incidental. She was administered bioavailable silicon as Biosil™ in solid form and magnesium, in the form of Promagnor™. The daily doses were 10 mg Silicon (elemental weight) and 450 mg Magnesium (elemental weight). The choline stabilizer was therewith administered at a daily dose of 200 mg. The incidental cramps during running did not occur again.

### EXAMPLE 7

A 75-year old person employed as a journalist was a kidney patient, undergoing dialysis three times per week, for four hours. Muscle cramps occurred during the day, at least once or twice a week, and particular in the hands, which made writing cumbersome. He was administered bioavailable silicon as Biosil™ in solid form and magnesium, in the form of Promagnor™ The daily doses were 10 mg Silicon (elemental weight) and 450 mg Magnesium (elemental weight). The choline stabilizer was therewith administered at a daily dose of 200 mg. The cramps disappeared.

So, in summary, a pharmaceutical composition is provided that includes bioavailable silicon and bioavailable magnesium. The bioavailable silicon is preferably orthosilicic acid and/or oligomers thereof that is combined with a stabilizing agent for inhibition of polymerisation of orthosilicic acid. Most preferably, the stabilizing agent is a choline compound. The pharmaceutical composition can also be in the form of a combination of two or more separate formulations. Suitably, the said pharmaceutical composition and/or the formulations of the combination are both for oral administration. However, rectal administration is not excluded. In case of the use of separate formulations, both formulations may be administered at the same time, but can also be administered at different times during the day. In case of the use of separate formulations, it is preferred that a first formulation contains the silicon compound and a second formulation contains the bioavailable magnesium. However, it is not excluded that a first formulation comprises both bioavailable silicon and bioavailable magnesium, and a second formulation comprises one of bioavailable silicon and bioavailable magnesium. The use of the said pharmaceutical composition and the use of said pharmaceutical combination is deemed advantageous both in a method for prevention of muscle cramps, such as skeletal muscle cramps, and in a method for the inhibition and/or treatment of muscle cramps, such as skeletal muscle cramps. The use for the prevention of skeletal muscle cramps may include the administration prior to performance of a muscular activity, such as sports, running, yoga, competitive activities, such as exams, sport competition; travelling by car, bicycle and/or motor, more particularly during rush hours, Daily doses of bioavailable magnesium are in the range of 50-500 mg/day, preferably 50-200 mg/day for the prevention and 200-500 mg/day for the treatment of existing and recurrent skeletal muscle cramps. For emergency cases, a dose of 400-500 mg/day may be provided. The specified amount is on the basis of elemental magnesium as orally administered. Daily doses of bioavailable silicon are 3-20 mg/Si. For the prevention, a dose of up to 12 mg/day Si, such as up to 10 mg/day Si is preferred. For the treatment, a dose of at least 10 mg/day Si is preferred. The doses are herein specified on the basis of elemental silicon as orally administered.

## Claims

1. A pharmaceutical composition of matter comprising a pharmaceutically effective amount of bioavailable silicon, and a pharmaceutically effective amount of a bioavailable magnesium compound for use in prevention, inhibition or treatment of muscle cramps.

2. The pharmaceutical composition as claimed in claim 1, wherein the combination is for use in prevention, inhibition or treatment of recurrent and/or idiopathic muscle cramps.

3. The pharmaceutical composition as claimed in any of the claims 1-2, wherein the muscle cramps comprise skeletal muscle cramps and/or smooth muscle cramps.

4. The pharmaceutical composition of claim 3, wherein the skeletal muscle cramps are cramps in the extremities, such as in leg, foot and hand.

5. The pharmaceutical composition of claim 3, wherein the smooth muscle cramps are cramps of digestive tract smooth muscles, such as oesophageal, stomach, and intestinal smooth muscles, or cramps of cervical smooth muscle.

6. The pharmaceutical composition as claimed in any of the claims 1-5, wherein silicon and magnesium are concomitantly, separately, or time-delayed administered orally or parenterally.

7. The pharmaceutical composition as claimed in any of the claims 1-6, wherein silicon and magnesium are administration on a daily basis.

8. The pharmaceutical composition any of the claims 1-7, wherein the pharmaceutically effective amount of bioavailable silicon is in a dose at least 3 mg/day elemental silicon, preferably at least 5 mg/day, more preferably at least 8 mg/day, and the pharmaceutically effective amount of a magnesium compound is in a dose 50-600 mg/day elemental magnesium, preferably in a dose of 100-500 mg/day.

9. Pharmaceutical composition of matter comprising a pharmaceutically effective amount of bioavailable silicon corresponding to a daily dose of at least 3 mg elemental silicon, preferably at least 5 mg, more preferably at least 8 mg, and a pharmaceutically effective amount of a magnesium compound corresponding to a daily dose of 50-500 mg elemental magnesium, preferably a daily dose of 100-450 mg, such as 200-400 mg.

10. The pharmaceutical composition of any of the claims 1-9, wherein the pharmaceutical composition of matter is a pharmaceutical combination of separate dosage forms or wherein the bioavailable silicon and bioavailable magnesium compound are part of a single pharmaceutical formulation.

11. Pharmaceutical composition of matter as claimed in any of the claim 1-10, wherein the bioavailable silicon is a silicon compound of the formula YₓSi(OH)₄₋ₓ or an oligomer thereof, wherein Y is optionally substituted (C₁-C₄)alkyl, (C₂-C₅)-alkenyl, (C₁-C₄)-alkoxy, amino, and wherein x is 0-2, wherein preferably the silicon compound is chosen from the group of orthosilicic acid or an oligomer thereof (x=0 in the formula), and mono(C₁-C₄)alkyl-trisilanol and combinations thereof, and more preferably the silicon compound is orthosilicic acid or an oligomer thereof.

12. Pharmaceutical composition and the pharmaceutical composition as claimed in claim 11, wherein the silicon compound is provided with a stabilizing agent inhibiting polymerisation of the silicon compound, wherein the stabilizing agent is preferably chosen from the group of amino acids, peptides and protein hydrolysates, organic acids, phenol and polyphenolic compounds, polyalcohols such as maltodextrine, quaternary ammonium compounds and aldehydes, preferably wherein the stabilizing agent is or comprises a quaternary ammonium compound, and wherein most preferably the stabilizing agent is choline.

13. Pharmaceutical composition as claimed in any of the claim 1-12, wherein the magnesium is provided for oral administration as a magnesium salt, such as an inorganic magnesium salt and/or an organic magnesium salt, preferably an organic magnesium salt, more preferably at least two organic magnesium salts.

14. Pharmaceutical composition of any of the preceding claims, wherein the silicon compound is further provided with a binder material, said combination of silicon compound, stabilizing agent and binder being in granulate form, wherein the magnesium is in solid form and the granulate and the magnesium are present in the pharmaceutical formulation as a solid mixture.

15. Pharmaceutical composition as claimed in claim 14, wherein the binder is a water-soluble binder material, for instance a cold-water soluble starch.
